# EUROPÄISCHE PATENTSCHRIFT

(11) **EP 1 074 540 B1**
(45) Veröffentlichungstag und Bekanntmachung des Hinweises auf die Patenterteilung: **07.05.2003**
(21) Anmeldenummer: 99115528.4
(22) Anmeldetag: 05.08.1999
(51) Int. Cl.: C07C 51/43, C07C 51/34, C07C 51/41, C07C 51/487, C07C 55/02, C07C 55/18, C07C 231/12, C07C 231/24, C07C 233/36

(54) **Verfahren zur Gewinnung von gesättigten Dicarbonsäuren aus der Ozonolyse ungesättigter Fettsäuren**
Process for the recovery of saturated dicarboxylic acids from the ozonolysis of unsaturated fatty acids
Procédé pour l'obtention d'acides dicarboxyliques saturés par ozonolyse d'acides gras unsaturés

(43) Veröffentlichungstag der Anmeldung: 07.02.2001
(73) Patentinhaber: Dr. Frische GmbH, D-63755 Alzenau (DE)
(72) Erfinder: Volkheimer, Jürgen, Dr., 63450 Hanau (DE); Frische, Rainer, Dr., 60529 Frankfurt am Main (DE); Hegwein, Katja, 64354 Reinheim/Odenwald (DE)
(74) Vertreter: Lippert, Marianne

(56) Entgegenhaltungen:
- US-A- 2 813 113
- US-A- 3 402 108
- PRYDE E H ET AL: "Reaction of azelaaldehydic esters" JOURNAL OF THE AMERICAN OIL CHEMISTS' SOCIETY, Bd. 46, Nr. 4, April 1969 (1969-04), Seiten 213-8, XP002128006

## Beschreibung

Die Erfindung betrifft ein Verfahren zur Gewinnung von für die Polymer- oder Polyamidsynthese geeigneten Dicarbonsäuren gemäß dem Oberbegriff des Patentanspruchs 1.

Die zur Gewinnung von Dicarbonsäuren angewandte Fettsäurespaltung ungesättigter nativer Fettsäuren zu Di- und Monocarbonsäuren ist allgemein bekannt. Das in den einschlägigen Chemiewerken als bekanntestes Beispiel hierfür angeführte Verfahren ist die oxidative Ozonolyse von Ölsäure zu Pelargonsäure und Azelainsäure. Bei der oxidativen Ozonolyse werden die ungesättigten Fettsäuren mit Ozon zu Ozoniden umgesetzt und diese direkt, d.h. ohne sie zu isolieren, oxidativ weiter zu den Carbonsäuren umgesetzt. Auf diese Weise läßt sich z.B. die Ölsäure (C18:1-Carbonsäure) in Pelargonsäure (C9-Monocarbonsäure) und Azelainsäure (C9-Dicarbonsäure) spalten.

Weiterhin ist bekannt, daß die beschriebene, zweistufige Reaktion bei ungesättigten, nativen Fettsäuren, insbesondere Ölsäure, vorteilhaft in Pelargonsäure als Lösemittel und in Anwesenheit von Wasser durchgeführt wird. Die Gewichtsmengenverhältnisse Fettsäure zu Lösemittel betragen dabei etwa 1:1. Die Menge an Wasserzusatz ist abgängig von der Art, wie die jeweilige Prozeßführung die Prozeßwärme abführt. Die Verwendung von Pelargonsäure ist deshalb vorteilhaft, weil diese ohnehin im Prozeß entsteht. Der Zusatz von Wasser verhindert zum einen bei der Ozonolyse die Bildung unerwünschter Nebenprodukte, zum anderen dient Wasser bei der Oxidation der Doppelbindung der Fettsäure zum Ozonidring und bei dessen oxidativer Spaltung zu Carbonsäuren als Reaktionswärme aufnehmendes und abführendes Medium.

Die Umsetzung einfach ungesättigter Fettsäuren wie Ölsäure mit Ozon wie auch die Aufreinigung der bei der oxidativen Ozonolyse entstehenden Folgeprodukte, wie z.B. Pelargonsäure und Azelainsäure bei der Ölsäure, gestalten sich schwierig. Hauptgründe hierfür sind:
1. Als Reaktionskomponenten werden nicht reine Verbindungen wie z.B. reine Ölsäure eingesetzt sondern Konzentrate, in denen die umzusetzende Komponente lediglich mehr oder weniger angereichert ist. So wird in der Produktionstechnik Ölsäure als 70%-iges bis 80%-iges Konzentrat (Gewichtsprozent des bei der Spaltung gewünschten Typs der einfach ungesättigten Ölsäure bezogen auf die Gesamtmenge) eingesetzt, das gesättigte, andere einfach sowie mehrfach ungesättigte Fettsäuren enthält.
2. Da die oxidative Ozonolyse die Ausgangsverbindung in zwei, z.B. bei der Ölsäure etwa gleich große, Bruchstücke zerlegt, ändert sich das Verhältnis von Verunreinigungen zu gewünschtem Stoff zu Ungunsten der Folgeverbindungen. Enthält 70%iges Ölsäurekonzentrat z.B. 10 Gew.% gesättigte Fettsäuren und ist damit das Gewichtsverhältnis Ölsäure zu gesättigten Fettsäuren 7/1, so ist nach der Umsetzung das Gewichtsverhältnis Azelainsäure und Pelargonsäure zu gesättigten Fettsäuren jeweils etwa 3,5/1. Die Isolierung der reinen Folgeprodukte in hoher Ausbeute wird dadurch erschwert.
3. Als äußerst reaktives Oxydationsmittel kann Ozon auch andere Komponenten der Reaktionslösung als die gewünschten Reaktionskomponenten angreifen. Dies gilt auch fiir die verfahrensgemäß bereits zu Ozonid umgesetzte Hauptkomponente der Reaktionslösung.
4. Die Reaktion des Ozons mit Doppelbindungen führt über eine Reihe von höchst instabilen und reaktiven Zwischenprodukten (vgl. "Organikum", VEB Deutscher Verlag der Wissenschaften, achte Auflage, 1968, Seite 252). Diese reaktiven Zwischenprodukte können zu unerwünschten Nebenprodukten, wie Persäuren, Perestern und -ethern reagieren.
5. Mehrfach ungesättigte Fettsäuren reagieren in der oxidativen Ozonolyse unter Bildung von kurzkettigeren Mono- und Dicarbonsäuren. Dies erschwert die Isolierung reiner Folgeprodukte.
6. Mehrfach ungesättigte Fettsäuren enthalten aktive Methylengruppen, die insbesondere nach oxidativem Angriff zu unerwünschten Nebenreaktionen wie z.B. Verschieben der Doppelbindungen, Vernetzung mit anderen Fettsäuren und Polymerisation führen.

In der industriellen Produktionstechnik findet die oxidative Ozonolyse ungesättigter Fettsäuren hauptsächlich Anwendung auf Ölsäurekonzentrat aus Talg. Pelargonsäure dient dabei als Reaktionslösemittel, wobei hauptsächlich Azelainsäure als Dicarbonsäure und Pelargonsäure als Monocarbonsäure entstehen. Durch geeignete Wahl der Reaktionsbedingungen lassen sich die unerwünschten Neben- bzw. Folgeprodukte minimieren. So wird die oxidative Ozonolyse von Ölsäurekonzentrat in zwei Stufen durchgeführt, wobei zunächst die eigentliche Ozonolyse durch Anlagerung des Ozons an die Doppelbindung mit Umlagerung zum Ozonidring bei Temperaturen < 50° C in einem ersten Reaktor durchgeführt wird. Zur Vermeidung von unerwünschten Nebenreaktionen wird die Reaktionswärme durch intensiven Wärmeaustausch abgeführt. Die Reaktion wird deshalb in Anwesenheit von Wasser als Wärme aufnehmendes und abführendes Medium durchgeführt. Die Oxidation des Ozonidringes mit Sauerstoff erfolgt dann bei ca. 100° C in einem zweiten Reaktor. Die organische Phase und die Wasserphase werden dabei mit dem gasförmigen Sauerstoff in intensiven Kontakt gebracht. Auch hier wird zur Vermeidung unerwünschter Nebenreaktionen die frei werdende Reaktionswärme über einen intensiven Wärmeaustausch mit Wasser abgeführt.

Mit einer zweistufigen Prozeßführung erreicht man bezogen auf die eingesetzte Ölsäure eine Ausbeute von > 90% der theoretisch gewinnbaren Menge an Pelargonsäure und Azelainsäure. Eine zweistufige oxidative Ozonolyse mit derartigen Ausbeuten ist z.B. in "Die Ozonolyse ungesättigter Fettsäuren", Dr. Martin Witthaus, Henkel KGaA, aus der Schriftenreihe des Fonds der chemischen Industrie, Heft 26, Frankfurt am Main 1986, dargelegt.

Die entstehenden Produkte, insbesondere die entstehende Dicarbonsäure Azelainsäure, werden im Stand der Technik in aufwendigen Reinigungsoperationen gewonnen. Die bei der Reaktion entstandene sowie auch die als Lösemittel eingesetzte Pelargonsäure kann destillativ zurückgewonnen werden. Als Vorlauf gehen dabei kürzerkettige Monocarbonsäuren über. Die entstandenen Dicarbonsäuren können im Falle der oxidativen Ozonolyse von Ölsäurekonzentrat aus dem Rückstand der Pelargonsäuredestillation über eine Hochtemperatur-Vakuumdestillation und/oder durch Elution mit Wasser gewonnen werden. Die so gewonnene Azelainsäure ist technisch rein, enthält jedoch noch Reste von Monocarbonsäuren und andere, insbesondere kürzer kettige Dicarbonsäuren.

Um Azelainsäure für die Polymersynthese zu gewinnen, muß die in technischer Reinheit gewonnene Azelainsäure weiter aufgereinigt werden. Zur Feinreinigung der Produkte können sowohl die jeweiligen Mono- wie auch die Dicarbonsäuremischungen, - vorteilhaft nach Überführung in Methylester -, einer fraktionierten Destillation unterworfen werden. Die so aufwendig gereinigte Azelainsäure mit einem Dicarbonsäuregehalt > 99 Gew.% wird für die Polyamidsynthese in die Diammoniumsalze von entsprechenden Diaminen überführt und kondensiert. Aufwendige Aufreinigungsverfahren der beschriebenen Art für Azelainsäure finden sich z.B. in der US 3 402 108.

Bei der oxidativen Ozonolyse von Erucasäure (C22:1), bei der Pelargonsäure und Brassylsäure entstehen, kann die Brassylsäure (C13-Dicarbonsäure) mit vertretbarem Aufwand weder direkt destillativ gewonnen noch mit Wasser eluiert werden. Hier verbleiben als klassische Reinigungsoperationen eine aufwendige und langwierige Umkristallisation aus geeigneten Lösemitteln wie Aceton, Acetonitril und Ethanol/Wasser sowie die Destillation der Ester (vorzugsweise der Methylester) der gewonnenen Säure.

Entsprechend ist im Stand der Technik eine Gewinnung reiner Brassylsäure über die Verfahrenskette 1. Veresterung der rohen Brassylsäure zu Methylester, 2. fraktionierte Vakuumdestillation des Methylesters und 3.Verseifen des reinen Methylesters üblich. Da in vielen Anwendungen der Methylester zur Anwendung kommt, kann in diesen Fällen auf die Darstellung der reinen Säure verzichtet werden. Bereits der erste Reinigungsschritt dieser Verfahrenskette über die Esterherstellung und fraktionierte Vakuumdestillation ist mit erheblichem Aufwand und Ausbeuteverlusten verbunden.

In der Literatur ist in "Reactions of Azelaaldehydic Esters", E.H. Pryde und J.C. Cowan, J. Am. Oil Chem. Soc., Band 46, 1969, Seiten 213 bis 218, die Ozonolyse von Dioleodiamiden bzw. Bis-Ölsäure-Diamiden beschrieben, wie sie aus Umsetzung von Diaminen, beispielsweise dem Ethylendiamin, mit Ölsäure erhältlich sind. Eine Durchführung des Verfahrens im technischen Maßstab ist nicht bekannt geworden. Das Bis-Ölsäure-Diamid läßt sich durch oxidative Ozonolyse in die jeweilige diamidische Dicarbonsäure Diazelaindiamid bzw. Bis-Azelainsäure-Diamid überführen, in deren vorteilhaft langkettiger Struktur die NH-Gruppen eingebunden sind, wie z.B. aus dieser letztgenannten Literaturstelle entnehmbar ist. Diese diamidische Dicarbonsäure stellt insbesondere für die Bildung von Polyamiden einen interessanten Polymerbaustein dar. Das Bis-Erucasäure-Diamid sollte sich in analoger Weise zu dem ebenfalls interessanten Baustein Bis-Brassylsäure-Diamid überführen lassen.

Die in Betracht gezogene Verwendung der erzeugten diamidischen Dicarbonsäure Bis-Azelainsäure-Diamid als Baustein für hochmolekulare Polymere setzt jedoch sehr hohe Reinheit voraus. Daher wurde nach der letztgenannten Literaturstelle versucht, das Filtrat des über nacht abgekühlten Reaktionsendprodukts der oxidativen Ozonolyse durch innigste wiederholte Durchmischung mit Ether aufzureinigen. Das über nacht stehen gelassene und abfiltrierte Reaktionsendprodukt enthielt im übrigen als Reaktionslösemittel Ameisensäure. Der für die gewonnenen Bis-Azelainsäure-Diamide in dieser Literaturstelle angegebene Schmelzbereich charakterisiert jedoch nach den Erkenntnissen der Erfinder der vorliegenden Anmeldung keine für den genannten Zweck ausreichend reinen Bausteine. Für ein ausreichend feingereinigtes Produkt müßte der Schmelzbereich höher liegen als angegeben.

Eine nicht ausreichende Aufreinigung hat allerdings gravierende Folgen. Soweit derartige diamidische Dicarbonsäuren nämlich aus Ölsäurekonzentrat erzeugt werden, entstehen stets neben den symmetrischen Dioleodiamiden auch gemischte Diamide, wie Stearo-Oleodiamid, die bei der oxidativen Ozonolyse zu monoreaktiven Diamidverbindungen gespalten werden. Letztere führen bei der Polymerbildung zu Kettenabbrüchen und damit zu einer erheblichen Eigenschaftsverschlechterung der entstehenden Polymere, wenn sie nicht hinreichend entfernt werden.

Der Erfindung liegt die Aufgabe zugrunde, ausgehend von den Verfahrensmerkmalen im Oberbegriff des Anspruchs 1 ein Verfahren zu schaffen, das es auf technisch möglichst einfache und kostengünstige Art gestattet, die gewünschten für die Polymer- oder Polyamidsynthese geeigneten gesättigten Dicarbonsäuren, zu gewinnen. Die Dicarbonsäuren sollen dabei gemäß dem Oberbegriffs des Anspruchs 1 sowohl nicht-diamidische Dicarbonsäuren, wie sie durch oxidative Ozonolyse ungesättigter Fettsäuren gewinnbar sind, als auch amidische Dicarbonsäuren, wie sie durch oxidative Ozonolyse der Bis-Fettsäurediamide der ungesättigten Fettsäuren gewinnbar sind, umfassen. (Wird im folgenden allgemein von Dicarbonsäuren gesprochen, so gelten die Aussagen sowohl für diamidische als auch nicht-diamidische Dicarbonsäuren).

Diese Aufgabe wird durch dasVerfahren zur Gewinnung von gesättigten Dicarbonsäuren der Kettenlänge C6 bis C21 bzw. entsprechenden diamidischen Dicarbonsäuren aus einer Fettsäurespaltung ungesättigter Fettsäuren bzw. der Bis-Fettsäurediamide dieser ungesättigten Fettsäuren durch oxidative Ozonolyse und nachfolgende Abtrennung und Aufreinigung der Dicarbonsäuren gelöst, das dadurch gekennzeichnet ist,
a) daß nach der oxidativen Ozonolyse deren Reaktionsprodukte in einer Carbonsäure oder einer Mischung mehrerer Carbonsäuren mittlerer Kettenlänge von C6 bis C12 oder von Estern kurzkettiger Alkohole dieser Carbonsäure(n) als Umkristallisationslösemittel in der Hitze gelöst werden,
b) daß die Lösung auf eine Temperatur abgekühlt wird, bei der die Dicarbonsäuren möglichst weitgehend auskristallisiert sind, jedoch das Umkristallisationslösemittel die übrigen Reaktionsprodukte der oxidativen Ozonolyse und anderen Verunreinigungen noch in gelöster Form enthält und selbst nicht ausfällt, und
   daß die auskristallisierten Dicarbonsäuren anschließend dadurch gewonnen werden,
c1 ) daß sie im noch abgekühlten Zustand in Form eines Kristallbreis abgetrennt werden, der noch Reste an dem Umkristallisationslösemittel mit den darin gelösten Bestandteilen enthält,
c2) der Kristallbrei gegebenenfalls nach weiteren Umkristallisationsschritten mit einem entsprechend abgekühlten Waschlösemittel oder einer Waschlösemittelmischung gewaschen wird und
c3) die Waschlösemittelreste anschließend entfernt werden.
Vorteilhafte Weiterbildungen des Verfahrens sind in den Unteransprüchen definiert.

Der gefundene Weg nach Anspruch 1 bedeutet ein Abgehen von der üblichen Praxis und sein Erfolg ist in mehrfacher Hinsicht überraschend.

Der erfindungsgemäße Einsatz der im Anspruch definierten mittellangen Carbonsäuren einer Kohlenstoffkettenlänge, wie sie der Kettenlänge der bei der oxidativen Ozonolyse von Fettsäuren entstehenden Carbonsäuren entspricht, als Umkristallisationslösemittel und vorzugsweise auch als Waschlösemittel, stellt sich dem Fachmann als nahezu abwegige Maßnahme dar.

So konnte er prinzipiell nicht erwarten, daß eine Umkristallisation, mit welchen Lösemitteln auch immer, schnell und unproblematisch zu den Produkten gewünschter Reinheit führen würde, da die abzutrennenden Verunreinigungen ein zu ähnliches Verhalten wie die zu gewinnende Verbindung aufweisen und zudem als langkettige Fettsäurederivate nach den bisherigen Erkenntnissen und Erfahrungen der Fachwelt dazu neigen, in auskristallisierende Verbindungen eingeschlossen zu werden. Eine Umkristallisation wäre deshalb erfahrungsgemäß vielfach zu wiederholen, wobei unweigerlich hohe Ausbeuteverluste eintreten würden.

So ist man bisher so vorgegangen, daß zunächst das Reaktionslösemittel zumeist in Form der bei der Reaktion ohnehin entstehenden mittellangen Monocarbonsäure (häufig Pelargonsäure) destillativ entfernt wird, um das zu lösende Trennproblem zu vereinfachen und damit zu erleichtern. Dieser Weg ist um so naheliegender, als es sich bei der üblicherweise als Reaktionslösemittel eingesetzten Pelargonsäure um eine Monocarbonsäure des Fettsäuretyps handelt, die bereits in geringen Mengen in der Mehrzahl der Anwendungen der Dicarbonsäuren und insbesondere bei der Synthese von Polymeren negative Auswirkungen hat. Danach wird die Dicarbonsäure destillativ gereinigt, wobei Reste von kurzkettigen Monocarbonsäuren und vor allem auch die Pelargonsäure sicher entfernt werden können. Daß nun ausgerechnet eine Zugabe von Pelargonsäure oder einer dieser ähnlichen Carbonsäure zusätzlich zu der nicht entfernten Menge Pelargonsäure in der Reaktionsendlösung der oxidativen Ozonolyse das Trennproblem vereinfacht und eine erfolgreiche Umkristallisation ermöglicht, konnte für den Fachmann nur überraschend sein. In diesem Zusammenhang sind auch die folgenden Umstände und Tatsachen beachtlich.

Üblicherweise werden als Lösemittel zur Umkristallisation leicht zu entfernende, niedermolekulare Verbindungen mit ausgeprägten, das Löseverhalten bestimmenden, chemischen Strukturmerkmalen eingesetzt. Zur Findung eines geeigneten Lösemittels werden dabei in der Regel gängige Lösemittel mit abgestufter Polarität geprüft. Typische Lösemittel sind z.B. leicht flüchtige Kohlenwasserstoffe (äußerst unpolar), leicht flüchtige, aromatische Kohlenwasserstoffe wie Benzol und Toluol, leichtflüchtige, halogenierte Kohlenwasserstoffe wie Chloroform und Trichlorethylen, Diethylether, Aceton, Essigester, Alkohole wie Methanol, Ethanol, Isopropanol, Acetonitrile, organische Säuren wie Ameisensäure und Essigsäure und viele mehr. Unter diesen Stoffen und vor allem Gemischen findet sich in der Regel ein für die Fest/Flüssigtrennung (z.B. für eine Umkristallisation) geeignetes Lösemittelsystem. Die in der Erfindung eingesetzten Fettsäuren bzw. Carbonsäuren und Carbonsäureester mittlerer Kettenlänge zählen nicht zu diesen üblichen Lösemitteln. Im Vergleich zu diesen sind sie weder leicht zu verdampfen, noch ist erkennbar, wie sie anderweitig wieder leicht aus dem gereinigten Produkt entfernbar sind. Darüber hinaus weisen sie kein erkennbares, für den Reinigungsprozeß besonders geeignetes Strukturmerkmal auf, das ihren Einsatz für den Reinigungsprozeß lohnend erscheinen ließe.

Üblicherweise liegen am Ende des oxidativen Ozonolyseprozesses die Reaktionsprodukte heiß (ca. 100°C) und hoch konzentriert gelöst in Pelargonsäure vor. Aus einer derartigen Lösung sollten die Dicarbonsäuren beim Abkühlen - wenn überhaupt - nur in äußerst unreiner Form mit einem hohen Anteil von eingeschlossenen Verunreinigungen auskristallisieren. Letzteres ist insbesondere deshalb zu erwarten, weil Dicarbonsäuren und abzutrennende Verunreinigungen gleichartige Strukturmerkmale aufweisen. Nach Entfernung der Mutterlauge sollte daher nur ein sehr unreines Produkt erhalten werden. Die zugehörige Mutterlauge sollte zudem aufgrund ihres relativ hohen Gehaltes an Verunreinigungen gleichartigen chemischen Aufbaus erhebliche Mengen an Dicarbonsäuren gelöst enthalten. Die Abtrennung der Mutterlauge hätte somit hohe Ausbeuteverluste zur Folge.

Die Erfinder konnten jedoch nachweisen, daß es mit den erfindungsgemäßen Umkristallisationslösemitteln gelingt, reine Dicarbonsäuren in hoher Ausbeute auszukristallisieren und daß, wenn man anschließend die am Kristallbrei anhaftende Mutterlauge der Umkristallisation mit kaltem Waschlösemittel auswäscht, auch verhindert werden kann, daß einerseits die Ausbeute durch Rücklösen der gefällten Verbindung zurückgeht und andererseits Verunreinigungen bei der Entfernung der Mutterlauge an den Dicarbonsäurekristallen verbleiben.

Überraschenderweise fallen die langkettigen Fettsäuren wie Palmitinsäure und Stearinsäure, die stets in den Rohstoffen enthalten sind und in dem Reaktionslösemittel in der Kälte sehr schlecht löslich sind, nicht mit aus. Geht man z.B. von Ölsäurekonzentrat als Rohstoff mit einem Stearinsäure- und Palmitinsäuregehalt von 10 Gew.% aus und wählt man das für die Ozonolyse gängige Ölsäurekonzentrat-/ Pelargonsäure Gewichtsmischungsverhältniss von 1:1, so erhält man nach der oxidativen Ozonolyse eine bei <25°C die Löslichkeit der Stearinund Palmitinsäure im Lösemittel Pelargonsäure übersteigende Konzentration. Beim Abkühlen der Reaktionslösung auf < 25°C sollten diese Säuren daher mit ausfallen.

Die Mutterlauge läßt sich zudem aus dem nach Abkühlen der Reaktionslösung entstehenden Feststoffbrei durch übliche Trennoperationen wie Filtrieren oder Zentrifugieren nur schwer abtrennen, denn die Filtriergeschwindigkeit ist niedrig und nimmt schnell durch Verdichtung der Feststoffmasse ab, die Poren des Filtermaterials setzen sich schnell zu und erhebliche Mengen an Mutterlauge verbleiben im Feststoff. Dieses Verhalten weist für den Fachmann in der Regel darauf hin, daß es sich entgegen der Feststellung der Erfinder bei der ausgefallenen Festphase nicht um ein einheitliches Produkt, sondern um ein Stoffgemenge handelt, mit der Schlußfolgerung, daß das verwendete Reaktionslösemittel sich nicht für die Reinigung des gesuchten Stoffes über eine Fest-Flüssigtrennung wie z.B. die Umkristallisation eignen kann.

Erfindungsgemäß werden als Umkristallisationslösemittel Carbonsäuren der mittleren Kettenlänge von C6 bis C12 oder die Ester kurzkettiger Alkohole dieser Carbonsäure(n) eingesetzt. Da häufig bei der oxidativen Ozonolyse von Fettsäuren gerade derartige Carbonsäuren als Reaktionslösemittel in Form der Pelargonsäure eingesetzt werden, die zudem auch bei der Reaktion entsteht, kann in diesen Fällen vorteilhaft die am Ende der Reaktion ohnehin zur Verfügung stehende Pelargonsäure als erfindungsgemäßes Umkristallisationslösemittel genutzt werden. Bevorzugt wird auch ein Ester der Pelargonsäure verwandt, der noch zusätzlich zugesetzt wird. Im einfachsten Fall kann man somit die etwa 100 °C heiße Ausgangslösung der oxidativen Ozonolyse abkühlen lassen, um die Auskristallisation zu bewirken. Benutzt man ein anderes Reaktionslösemittel für die oxidative Ozonolyse, das nicht einer im Anspruch 1 definierten Carbonsäure entspricht, wie Chloroform (vgl. die genannte Literaturstelle von Pryde und Cowan), so kann jedoch das Lösemittel auch entfernt und durch ein erfindungsgemäßes Umkristallisationslösemittel ersetzt werden. Andere geeignete Carbonsäuren als die Pelargonsäure sind Hexansäure, Heptansäure usw. bis zu einer Kettenlänge C12.

Die Abkühlungstemperatur liegt vorzugsweise unter 25 °C. Bei der Herstellung von amidischen Dicarbonsäuren sind jedoch auch höhere Temperaturen bis über 40 °C möglich, bei denen eine selektive Auskristallisation der gewünschten diamidischen Dicarbonsäuren in hoher Ausbeute erfolgt. Da der Vorgang leicht durchführbar und nachvollziehbar ist, kann der Fachmann den jeweils optimalen Temperaturwert leicht ermitteln, bei dem die Dicarbonsäuren möglichst weitgehend auskristallisiert sind, jedoch das Umkristallisationsmittel die übrigen Reaktionsprodukte der oxidativen Ozonolyse und anderen Verunreinigungen noch in gelöster Form enthält und selbst nicht ausfällt. Als Kontrolle hat sich hier die spektroskopische Analyse der Inhaltsstoffe der Mutterlauge bewährt, wobei z.B. eine gaschromatographische Untersuchung der veresterten Rückstände der Mutterlauge durchgeführt wurde.

Die auskristallisierten Dicarbonsäuren werden anschließend dadurch gewonnen, daß sie im noch abgekühlten Zustand in Form eines Kristallbreis durch Filtrieren, Dekantieren oder Zentrifugieren abgetrennt werden, wobei der Kristallbrei noch Reste an dem Umkristallisationsmittel mit den darin gelösten Bestandteilen enthält. Der Kristallbrei wird gegebenenfalls nach weiteren Umkristallisationsschritten mit einem entsprechend abgekühlten Waschlösemittel oder einer Waschlösemittelmischung vorzugsweise wiederum in Form der mittellangen Carbonsäuren bzw. deren Estern gewaschen, und anschließend werden die Waschlösemittelreste destillativ, alternativ durch Waschen z.B. mit Aceton oder Hexan, entfernt. Auf diese Weise konnten sowohl nicht-diamidische als auch diamidische Dicarbonsäuren in hoher Reinheit und guter Ausbeute gewonnen werden.

Bei der oxidativen Ozonolyse geht man vorzugsweise von einfach ungesättigten Fettsäuren nativen Ursprungs der Kettenlänge C14 bis C24 aus, z.B. aus Ölsäure, Erucasäure, Palmitoleinsäure, Myrestinoleinsäure, Eicosensäure, bzw. deren Diamiden aus, um zu möglichst hohen Ausbeuten zu gelangen. Prinzipiell ist das Verfahren jedoch auch mit mehrfach ungesättigten Fettsäuren durchführbar. Die oxidative Ozonolyse wird in bekannter zweistufiger Weise bevorzugt wiederum so durchgeführt, daß eine Ausbeute, wie im Stand der Technik, von über 85% der Theorie erzielt wird.

Um die Reaktionsprodukte der oxidativen Ozonolyse in der Hitze zu lösen, wendet man vorzugsweise eine Temperatur von nicht mehr als 100 °C an. Dies ist nicht nur ratsam, wenn man das Wasser der Reaktion im Reaktionsprodukt beläßt. Wenn man berücksichtigt, daß im zweiten Verfahrensschritt der oxidativen Ozonolyse ohnehin in der Regel mit 100 °C gearbeitet wird und man die bei dieser Temperatur auftretenden Nebenprodukte der Reaktion bewältigen muß, sollte man vermeiden, durch nachfolgend auf das Reaktionsprodukt angewandte höhere Temperaturen, etwaige weitere störende Begleitstoffe entstehen zu lassen. So können bei Temperaturen über 100 °C Zersetzungsprodukte der bereits vorhandenen Nebenprodukte und durch deren Polymerisation auch weitere störende und gegebenenfalls schwerer entfembare Begleitstoffe entstehen.

Vorzugsweise, speziell wenn man auf die weiter unten erläuterte weitere Aufreinigung durch Diammoniumsalzbildung verzichtet, werden die Waschlösemittelreste erst dann entfernt, wenn durch mehrmaliges Zusetzen einer bestimmten Waschlösemittelmenge oder durch sukzessives Durchdrücken einer Waschlösemittelsäule durch den Kristallbrei die am Kristallbrei anhaftenden Umkristallisationslösemittelreste im wesentlichen durch Waschlösemittel ersetzt sind.

Bevorzugt setzt man erfindungsgemäß danach die genannten mittellangen Carbonsäuren (von C6 bis C12, vorzugsweise der Kettenlänge C8 bis C10 insbesondere der Kettenlänge C9 ) und deren Ester kurzkettiger Alkohole wie Methyl-, Ethyl-, Propyl- und Isopropylester fiir Dicarbonsäuren der Kettenlänge (C6 bis C21) wie z.B. Azelainsäure (C9) und Brassylsäure (C13) als Umkristallisationslösemittel ein. Man arbeitet bei der Ozonolyse beispielsweise mit Pelargonsäure als Reaktionslösemittel und im übrigen so, daß bei der Ozonolyse und der oxidativen Ozonidringspaltung die Ausbeute an Dicarbonsäure mindestens 85 Gew.% der theoretisch zu erwartenden Ausbeute beträgt. Man sorgt ferner vorzugsweise dafür, daß keine gesättigten Fettsäuren einer Kettenlänge größer C28, bevorzugt größer C26 vorhanden sind und daß der Anteil gesättigter Fettsäuren mit Kettenlängen größer C14 und kleiner C26 unter 15 Gew.% , möglichst jedoch unter 10 Gew. % im Ausgangsgemisch der zu ozonisierenden Fettsäuremischungen liegt. Werden statt Fettsäuremischungen bei der oxidativen Ozonolyse Fettsäurediamidmischungen eingesetzt, so bedeutet dies, daß die in den Fettsäurediamiden gebundenen gesättigten Fettsäuren weniger als 15 Gew.%, vorzugsweise weniger als 10 Gew.% des Gesamtanteils der Fettsäuren ausmachen sollten. Im Fall nicht-diarnidischer Dicarbonsäuren setzt man ferner das Umkristallisationslösemittel bevorzugt mit dem ein- bis zehnfachen der nach Entfernung des Reaktionslösemittels Pelargonsäure zurückbleibenden Menge an Dicarbonsäuren, langkettigen Monocarbonsäuren wie z.B. Stearinsäure und Nebenprodukten der oxidativen Ozonolyse ein. Arbeitet man mit diamidischen Dicarbonsäuren als Endprodukt der oxidativen Ozonolyse, so empfiehlt sich stattdessen der Zusatz einer zwei- bis fünfzehnfachen Gewichtsmenge des Umkristallisationslösemittels. Im übrigen ist die obige Grenze des Zusatzes im Hinblick auf Handhabbarkeit und Ausbeute festzulegen. Prinzipiell kann man mit höheren Mengen arbeiten.

Man läßt ferner bevorzugt aus einer bei ca. 80°C gesättigten Lösung im Fall nicht-diamidischer Dicarbonsäuren (und aus einer bei ca. 100 °C gesättigten Lösung im Fall diamidischer Dicarbonsäuren) durch Abkühlen auf unter 25° C, vorzugsweise auf etwa 15° C, auskristallisieren und befreit die abgetrennte Kristallmasse möglichst vollständig von anhaftender Mutterlauge. Letzteres erfolgt durch Waschen mit kaltem (T kleiner 25°C vorzugsweise etwa 15°C) Lösemittel, wobei die jeweilige Abkühlungstemperatur im wesentlichen beibehalten wird. Das Waschen kann mit Fettsäuren und Fettsäuremischungen Fettsäurestern und Fettsäureestermischungen sowie Gemischen aus Säuren und Estern der oben für das Umkristallisationslösemittel angegebenen Kettenlängen erfolgen. Bei der Verwendung von Estern kann der untere Temperaturwert auch gesenkt werden, da diese tiefer schmelzen.

Nach vorzugsweise destillativer Entfernung der im erfindungsgemäß behandelten Feststoff verbleibenden Waschlösemittelreste wie z.B. Pelargonsäure und/oder Pelargonsäuremethylester verbleiben die Dicarbonsäuren wie z.B. Azelainsäure und Brassylsäure in hoher Reinheit zurück. Langkettige Monocarbonsäuren wie Palmitin- und Stearinsäure sowie unerwünschte Nebenprodukte der Ozonolyse sind mit den Wasch- bzw. Mutterlaugen abgetrennt. Im Falle der Verwendung von Mischungen der Lösemittelsäuren und/oder -ester kann das Lösemittel destillativ zurückgewonnen werden und, ohne in die Einzelkomponenten aufgetrennt zu werden, wiederverwendet werden.

Die produktionstechnisch einfachste Methode zur Abtrennung der als Waschlösemittel eingesetzten Fettsäuren und -ester ist deren destillative Entfernung, jedoch verbleiben im allgemeinen geringe Reste (1 bis max. 2 Gew.%) an Waschlösemittel im gereinigten Produkt. Für die Mehrzahl der Anwendungen sind diese Reste unbedeutend, nicht jedoch bei Verwendung der gereinigten Produkte für die Erzeugung von Polymeren.

Überraschenderweise kann die Abtrennung von Resten dieser Waschlösemittel und damit eine Feinreinigung der Dicarbonsäuren über die für die Polyamidsynthese übliche Überführung der Dicarbonsäuren in feste Diammonium-Dicarbonsäure-Salze vorzugsweise in alkoholischer Lösung beim isoelektrischem Punkt (ca. PH 7,5) erfolgen. Eine Reinigung ist dabei selbst bei Lösemittelrestgehalten im Kristallbrei deutlich über 2 Gew.% bis hin zu 50 Gew.% noch problemlos möglich. Dies ist um so überraschender, als gerade wegen der ähnlichen Kettenlängen ein Einschließen in die ausfallenden Salze zu erwarten ist. Die sich bildenden Ammoniumsalze der Reste der Waschlösemittelfettsäuren wie auch die Reste der Waschlösemittel-Ester verbleiben jedoch in der alkoholischen Mutterlauge. Die am Kristallbrei anhaftende Mutterlauge läßt sich durch Waschen mit Alkohol ohne nennenswerte Verluste vollständig entfernen. Die so gewonnenen Diammoniumsalze können vorteilhaft direkt für die Polyamidsynthese eingesetzt werden.

Überraschenderweise lassen sich darüber hinaus auch mitgeschleppte Restmengen der ursprünglichen Verunreinigungen wie langkettige, gesättigte Fettsäuren, Fettsäureester, -ether etc., wie sie bei nur unzureichender Waschung der Ausgangsfeststoffmasse gemäß Anspruch 1 zurückbleiben können, bei der Reinigung der Dicarbonsäuren über die Diammoniumsalze mit entfernen. Dies ergibt zusätzliche Produktionssicherheit und kann sogar vorteilhaft dadurch genutzt werden, daß der Wasch- und Waschlösemittelentfernungsschritt nach Anspruch 1 gezielt vorzeitig beendet wird, wenn die Endreinigung über das Diammoniumsalz durchgeführt werden soll. Der Aufwand für die Reinigungsoperationen läßt sich so erheblich verringern. Die Diamine stellen damit sowohl einen Hilfsstoff zur Reinigung insbesondere der nicht-diamidischen Dicarbonsäuren dar als auch eine Reaktionskomponente zur Erzeugung der Ausgangskomponenten zur Polyamidsynthese. Soweit sie als Hilfsstoff eingesetzt werden, können sie in an sich bekannter Weise zurückgewonnen und wiedereingesetzt werden. Prinzipiell kann jedoch auch bei den diamidischen Dicarbonsäuren über die Salzbildung aufgereinigt werden, wie weiter unten aufgezeigt ist.

Der nach destillativer Rückgewinnung der Lösemittel zurückbleibende Destillationssumpf enthält noch Reste an Dicarbonsäuren. Auch diese können zurückgewonnen werden, wenn man den Destillationssumpf in Alkohol aufnimmt und die Dicarbonsäuren als Diammoniumsalze ausfällt.

Durch Kombination der beiden oben beschriebenen Reinigungsoperationen, Waschung des aus dem Reaktionslösemittel ausgefallenen Dicarbonsäurefeststoffs und Fällung der Dicarbonsäure als Diammoniumsalz, lassen sich Ausbeuteverluste auf ein äußerst geringes Maß herabsetzen.

Bei dieser anschließenden Salzbildung kann man gemäß den Unteransprüchen 9 und 10 sowie Beispiel 4 prinzipiell auch im Anschluß an den Verfahrensschritt c1) von Anspruch 1 unter Auslassung der Schritte c2) und c3), oder nach Verwendung von Wasser als Waschlösemittel in den Verfahrensschritten c2) und c3) für eine bei der Oxidationsstufe verwendete Säure (z.B. Ameisensäure) direkt die Salzbildung anschließen. In diesem Fall sind allerdings in der Regel noch erhebliche Mengen an Pelargonsäure im Filterkuchen vorhanden (wie oben erwähnt bis 50 Gew.%) und man benötigt entsprechend große Mengen an Amiden für die Salzbildung. Daher erfolgt bevorzugt erst im Anschluß an den Verfahrensschritt c3) nach Waschen des Kristallbreis gemäß Schritt c2) vorzugsweise mit den im Anspruch 7 angegebenen Waschlösemitteln die Salzbildung.

Überraschenderweise lassen sich nach dem erfindungsgemäßen Verfahren auch Mischungen von Dicarbonsäuren, wie sie bei der oxidativen Ozonolyse von ungesättigten, nativen Fettsäuren mit unterschiedlicher Lage der Doppelbindungen entstehen, als reine, von Monocarbonsäuren und anderen bei der Ozonolyse entstehenden Verunreinigungen befreite (nicht-diamidische) Dicarbonsäuremischungen mit Kettenlängen >C6 bis <C21 gewinnen. So gewinnt man aus den durch Verseifen von erucareichen Raps- oder Crambeölen erhältlichen Fettsäuremischungen ein Gemisch von Azelainsäure und Brassylsäure, das frei von Monocarbonsäuren und unerwünschten Nebenprodukten der oxidativen Ozonolyse ist, so daß somit das Dicarbonsäuregemisch direkt zur Umsetzung zu Polyamiden eingesetzt werden kann. Eine Trennung der so erhaltenen Dicarbonsäuremischungen in die reinen Einzelkomponenten kann in an sich bekannter Weise erfolgen. Analoges gilt für die gemischt zusammengesetzten Dia-mide der entsprechenden ungesättigten Fettsäuren nach deren oxidativer Ozonolyse zu diamidischen Dicarbonsäuren.

Insbesondere eignen sich in diesem Zusammenhang Fettsäuremischungen als Rohstoff für die Gewinnung von Dicarbonsäuremischungen, wie sie durch Verseifen von durch Teilhydrierung von mehrfach ungesättigten Fettsäuren befreiten Ölen, insbesondere High-Oleic-Ölen, und Fischölen erhältlich sind. Aufgrund der Teilhydrierung und der durch sie bewirkten Verschiebung der Doppelbindungen erhält man ein ganzes Spektrum von Dicarbonsäuren unterschiedlicher Kettenlänge (von C6 bis C21). Auch in diesem Fall läßt sich nach der beschriebenen Methode die Dicarbonsäuremischung von die Polyamidsynthese störenden Nichtdicarbonsäureprodukten wie z.B. Monocarbonsäuren und Nebenprodukten der Ozonolyse befreien.

Die Erfindung gestattet somit in wesentlich einfacherer Weise als nach dem Stand der Technik, reine Dicarbonsäuren und Dicarbonsäuremischungen über die oxidative Ozonolyse zu erhalten.

Überraschenderweise hat sich nun ferner gezeigt, daß die in der obigen Literaturstelle "Reactions of Azelaadehydis Esters" aufgezeigten, für die Polymerherstellung interessanten Dicarbonsäurediamidtypen oder besser amidischen Dicarbonsäuren in hoher Ausbeute und in hoher Reinheit ebenfalls auf die im Anspruch 1 und den Unteransprüchen gekennzeichnete Weise analog gewonnen werden können. Vorzugsweise wird hier folgendermaßen vorgegangen: Die Ozonolyse der Diamide der Fettsäuren wird hierbei in Carbonsäuren einer Kettenlänge C7 bis C12, vorzugsweise (Pelargonsäure) C9, als Reaktionslösemittel durchgeführt, wobei keine klare Lösung vorhanden sein muß. Man sorgt durch geeignete Lösemittelmenge und Temperatur dafür, daß eine rührfähige Reaktionsmasse entsteht. Die Reaktion wird hierzu bei erhöhten Temperaturen um 60°C bis zu 80 °C in Anwesenheit von Wasser thermostatisiert durchgeführt, bevor der zweite Schritt der oxidativen Ozonolyse folgt.

Die Reaktionsprodukte der oxidativen Ozonolyse werden im Reaktionslösemittel nach Erwärmen auf 100°C vollständig gelöst und durch Abkühlen kristallisiert, vom Reaktionslösemittel abgetrennt und mit den oben genannten kalten Waschlösemitteln gewaschen.

Das anhaftende Waschlösemittel wird weitgehend z.B. destillativ entfernt und der Rückstand unter Verwendung von Alkohol als Lösemittel in Salze solcher Diamine überführt, die für die Polyamidherstellung Verwendung finden. Hierzu zählen vorzugsweise endständige Aminogruppen enthaltende, lineare Kohlenwasserstoffketten der Kettenlänge C4 bis C22.

Monocarbonsäuren und die in der Ozonolyse entstehenden Nebenprodukte lassen sich auf diese Weise bereits im ersten Kristallisationsschritt weitgehend entfernen. Im nachfolgenden Schritt der Überführung der Dicarbonsäuren in die Ammoniumsalze der Diamine, lassen sich bei geeigneter Wahl des Reaktionslösemittels die diamidischen Dicarbonsäuren als Ammoniumsalze auskristallisieren, während die noch vorhandenen Reste an Monocarbonsäuren in Lösung verbleiben. Dasselbe gilt, wie bereits aufgezeigt, auch für die über die Salzbildung aufzureinigenden nicht-diamidischen Carbonsäuren (Anspruch 9). Aus diesem Grunde stimmen die im folgenden dargelegten Kriterien für die Salzbildung für beide Fälle überein, nämlich für die Aufreinigung nicht-amidischer Carbonsäuren über die Salzbildung und für die Salzbildung der diamidischen Carbonsäuren für die Polyamidsynthese.

Als Reaktionslösemittel für die Salzbildung haben sich wasserlösliche Alkohole oder auch Alkoholmischungen bewährt (Methanol, Ethanol und Propanole), die vorzugsweise in der fünf- bis zehnfachen Gewichtsmenge zu der stöchiometrischen an Dicarbonsäure und Diamin zugegeben werden.

Durch Zusatz geringer Mengen an Wasser kann die Löslichkeit des Salzes so beeinflußt werden, daß aus einer klaren, heißen Lösung beim Abkühlen lediglich die Dicarbonsäuren bzw. die diamidischen Dicarbonsäuren als Salz auskristallisieren. Die Salze lassen sich mit den entsprechenden, kalten Alkohol/Wasser-Mischungen waschen und somit von anhaftender Mutterlauge befreien.

Über die Wahl des salzbildenden Diamins kann ebenfalls Einfuß auf die Löslichkeit des Salzes genommen werden. So sind die Salze aus Azelainsäure und kurzkettigen Diaminen relativ gut löslich, wohingegen deren mit längerkettigen Diaminen sich bildenden Salze schwerer löslich sind (Anspruch 12).

Die gebildeten Salze der diamidischen Dicarbonsäuren lassen sich, gegebenenfalls nach Umkristallisieren in Alkohol aus den entsprechenden Alkohol/Wasser-Mischungen und Trocknung direkt zur Polyamidsynthese einsetzen.

Die Dicarbonsäuren können auch in üblicher Weise mit verdünnten Mineralsäuren freigesetzt, vom sich bildenden Diammoniumsalz der Mineralsäuren durch Waschen befreit und in reiner Form gewonnen werden. Die entsprechenden Diamine wiederum können durch Neutralisation der Mineralsäuren in bekannter Weise zurückgewonnen werden.

Die Diammoniumsalzherstellung kann folgendermaßen erfolgen:
Die Reaktionsprodukte der oxidativen Ozonolyse mit amidischen oder nicht-amidischen Dicarbonsäuren werden durch Erwärmen im Reaktionslösemittel gelöst, die heiße Lösung gegebenenfalls mit heißem Wasser gewaschen, die vom Waschwasser befreite organische Lösung ausgefällt und über Filtration abgetrennt. Der so erhaltene, pelargonsäurehaltige, feste Rückstand wird, - vorzugsweise nach Waschen mit kaltem Aceton mit etwa stöchiometrischer Menge an entsprechendem Diamin versetzt, diese Mischung mit der fünffachen bis zehnfachen Gewichtsmenge an absolutem Alkohol bis zum Siedepunkt erhitzt und, soweit keine klare Lösung entsteht, wird in der Siedehitze so viel Wasser zugeben, bis eine klare Lösung entsteht. Der PH-Wert dieser Lösung wird entweder durch Zugabe von roher Dicarbonsäure oder Diamin auf ca. 7,5 gestellt und die Lösung auf Raumtemperatur abgekühlt.

Im folgenden wir die Erfindung anhand einiger Beispiele näher erläutert. Für alle Beispiele wurde die oxidative Ozonolyse der Säuren bzw. deren Diamide in bekannter Weise zweistufig und mit Ausbeuten über 85 % ausgeführt. Die Ausbeute wurde anhand der analysierten aliquoten Teile zurückgerechnet und kontrolliert.

### Oxidative Ozonolyse für das Ausgangsmaterial

### I. Erucasäure zu Pelargonsäure und Brassylsäure (Beispiele 1 bis 3)

100 g Erucasäure (Durchschnittszusammensetzung: 91,5 Gew.% C22:1, 1,5 Gew.% C22:2, 0,4 Gew.% C18:1, 0,4 Gew.% C18:2, 6 Gew.% gesättigte, langkettige Fettsäuren) wurden mit 100 g Pelargonsäure als Reaktionslösemittel und Wasser als Hilfsstoff in üblicher Weise zweistufig ozonisiert und oxidiert. Für die Oxidationsstufe wurde Ameisensäure mit Wasserstoffperoxid eingesetzt. Nach der Theorie sollten im Reaktionslösemittel enthalten sein:
67,1 g entstandene Brassylsäure (mit sehr wenig anderen Dicarbonsäuren)
43,8 g entstandende Pelargonsäure (mit sehr wenig mittellangen Monocarbonsäuren)
6,0 g nicht umgesetzte gesättigte langkettige Fettsäuren des Ausgangsrohstoffs

Nach der Oxidationsstufe lag das Oxidationsprodukt in 100°C heißer organischer Phase (Pelargonsäure mit darin gelöster Ameisensäure und Wasser) klar gelöst vor. Das Reaktionsgemisch wurde auf 20°C abgekühlt. Die Oberphase verfestigte sich dabei vollständig zu einem weißen Kristallbreikuchen. Die wässrige Phase wurde abgetrennt und verworfen. Der Kristallbreikuchen wurde verrührt und der dabei entstehende Brei zweimal mit 20°C kaltem Wasser gewaschen und jeweils mit Büchnertrichter und Schwarzbandfilter scharf abgesaugt. Die gewonnenen Filtrate der Waschung, die jeweils eine klare Wasserunterphase und eine klare Reaktionslösemitteloberphase (Pelargonsäure) zeigten, wurden gesammelt und vereinigt. Der zurückbleibende Filterkuchen wurde, wie unten angegeben, analysiert.

### Beipiel 1

### 1. Analyse des Rückstandes im abgetrennten Reaktionslösemittel

Ein Aliquot (7 g) der Mutterlauge, d.h. der Reaktionslösemitteloberphase wurde im Vakuum destillativ von flüchtigen Bestandteilen befreit. Es destillierten 5,95 g Pelargonsäure über. Als nicht flüchtige Bestandteile verblieben 0,65 g. Das Gaschromatogramm der zu Methylestern umgesetzten, nicht flüchtigen Bestandteile zeigt 30% Brassylsäuremethylester und 70% Fettsäureester.
Interpretation:
Bezogen auf den Gesamtansatz enthält die Reaktionslösemittelphase gelöst (0,65/5,95)(100 + 43,8) g =15,7 g nicht flüchtigen Rückstand. Darin sind langkettige, gesättigte Fettsäuren, Nebenprodukte und nicht ausgefallene Brassylsäure (nach Gaschromatogramm ca. 4,5 g) enthalten.

### 2. Analyse der rohen Brassylsäure aus dem Filterkuchen des kalten Reaktionslösemittels

Ein Aliquot (8,5 g) des Filterkuchens wurde im Vakuum destillativ von flüchtigen Bestandteilen befreit. Es destillierten 3,7 g Pelargonsäure (mit sehr wenig anderen mittellangen Monocarbonsäuren) über. Es verblieb ein Rückstand von 3,9 g (im folgenden als rohe Brassylsäure bezeichnet).

Der Rückstand hatte einen Schmelzbereich von 107,5°C bis 109,6°C. (Der in der Literatur aufgefundene tiefste Fusionspunkt Fp reiner Brassylsäure beträgt 112°C bis 113°C). Die Neutralisations-Titration des Rückstands ergab eine Abweichung gegenüber reiner Brassylsäure von ca. 0,5%.

### Beispiel 2

Ein Aliquot (20 g) des mit Wasser gewaschenen und scharf abgesaugten Filterkuchens wurde mit 45 g kalter Pelargonsäure bei 15°C einmal innigst verrührt und scharf abgesaugt. Es wurden 12 g Filterkuchen und 48 g Mutterlauge erhalten.

### 1. Analyse der Mutterlauge

Ein Aliquot (7 g) der Mutterlauge wurde im Vakuum destillativ von flüchtigen Bestandteilen befreit. Als nicht flüchtige Bestandteile verblieben 0,2 g. Das Gaschromatogramm der zu Methylestern umgesetzten, nicht flüchtigen Bestandteile zeigte die gleiche Zusammensetzung wie das des Rückstands der ersten Mutterlauge.

### 2. Analyse der gereinigten Brassylsäure aus dem Filterkuchen der kalten Pelargonsäure

Ein Aliquot (6,4 g) des mit Pelargonsäure gewaschenen Filterkuchens wurde im Vakuum destillativ von flüchtigen Bestandteilen befreit. Es destillierten 2,21 g Pelargonsäure über. Als Rückstand verblieben 3,6 g. Der Rückstand (gereinigte Brassylsäure) hatte einen Schmelzbereich von 110,5°C bis 112°C. (Lit.-Fp. reiner Brassylsäure 112°C bis 113°C). Die Neutralisations-Titration des Rückstands ergab eine Abweichung gegenüber reiner Brassylsäure von < 0,5%.

Interpretation: Durch kaltes Waschen findet keine Rücklösung der Brassylsäure statt. Es werden lediglich Mutterlaugenreste mit den in ihr gelöst enthaltenen Stoffe verdünnt und ausgewaschen. Durch einmaliges Waschen konnte die Brassylsäure in praktisch reiner Form gewonnen werden.

### Beispiel 3

### Reinigung von roher Brassylsäure über Hexamethylendiammoniumsalz

12,4 g rohe Brassylsäure, die analog zu Beispiel 1 hergestellt wurde, mit Resten an Pelargonsäure wurden in 20 g techn. Ethanol (5% Wassergehalt) in der Hitze gelöst (Lösung 1). Parallel wurden 5,5 g Hexamethylendiamin in 20 g techn. Ethanol gelöst (Lösung 2). Zu Lösung 1 wurde in der Hitze (bei ca. 70°C) unter Rühren Lösung 2 zugegeben, bis der PH-Wert der Mischung 7,5 betrug. Es wurde auf Raumtemperatur abgekühlt. Die entstehende feste Kristallbreimasse wurde verrührt und durch Filtrieren von der Mutterlauge soweit als möglich getrennt.

Es wurden 22 g gelbe Mutterlauge gewonnen, aus der nach Entfernen des Ethanols 0,54 g wachsartige Masse als Rückstand verblieben. Das in diesem Fall erstellte Dünnschichtchromatogramm zeigt neben Brassylsäure mehrere Verunreinigungen.

Die durch Filtration von der Mutterlauge weitgehend befreite, verbleibende Kristallmasse wurde mit 40 g heißem Ethanol verrührt, anschließend abgekühlt, vom Waschethanol durch Filtration weitgehend befreit und getrocknet. Es verblieben 16,5 g weißes Pulver. Das Dünnschichtchromatogramm zeigt Brassylsäure ohne Pelargonsäure. Das Waschethanol (ca. 40g) enthielt 0,45 g wachsartigen Rückstand. Das Dünnschichtchromatogramm zeigte neben Brassylsäure mehrere Verunreinigungen.

Es sei angemerkt, daß vor Erstellung der jeweiligen Dünnschichtchromatogramme zum Nachweis der Brassylsäure das gewonnene Salz wieder in die Säure umgesetzt wurde.

### Interpretation:

Der Versuch beweist, daß sowohl die nach der oxidativen Ozonolyse in roher Brassylsäure enthaltenen Nebenprodukte sowie auch Reste des Reaktionslösemittels Pelargonsäure ohne nennenswerte Verluste über das Diammoniumsalz abgetrennt werden können.

### II. Oxidative Ozonolyse für das Ausgangsmaterial des Beispiels 4

### Bis-Eruca-Ethylendiamid zu Bis-Brassylsäure-Ethylendiamid und Pelargonsäure

Die Umsetzung erfolgte analog zur oxidativen Ozonolyse, wie unter I aufgeführt mit Bis-Eruca-Ethylendiamid als Rohstoff, wobei allerdings die Ozonolyse in Pelargonsäure bei einer erhöhten Temperatur von etwa 60 °C durchgeführt wurde. Nach Abkühlen des heißen Oxidationsprodukts und zweimaligem Waschen mit Wasser wurde wie bei I filtriert bzw. abgesaugt und der zurückbleibende Filterkuchen gewonnen.

### Beispiel 4

### Reinigung von pelargonsäurehaltigem Bis-Brassylsäure-Ethylendiamid über Ethylendiammoniumsalz

15,7 g des pelargonsäurehaltigen (50 Gew.%) und das Bis-Brassylsäure-Ethylendiamid der Reaktion enthaltenden Filterkuchens aus II wurden mit 3,6 g Hexamethylendiamin in 450ml Ethanol umgesetzt. Es fielen 7,5 g des Diammoniumsalzes des Bis-Brassylsäure-Ethylendiamids aus. Nach dem erstellten Dünnschichtchromatogramm waren im Salz nur Spuren von Pelargonsäure enthalten. Der Rückstand der Mutterlauge (10,9 g) enthielt Pelargonsäure (als Salz) und Verunreinigungen.

### III. Oxidative Ozonolyse für das Ausgangsmaterial des Beispiels 5

### Ölsäure zu Azelainsäure und Pelargonsäure

Die Umsetzung erfolgte analog zur oxidativen Ozonolyse, wie unter I aufgeführt, allerdings wurde als Rohstoff High-Oleic-Sonnenblumensäure (90 Gew.% Ölsäure, 3 Gew.% Linolsäure, 6 Gew.% Palmitin- und Stearinsäure, und 1 Gew.% Fettsäuren mit Kettenlängen >C20) eingesetzt.
Nach Abkühlen des heißen Oxidationsprodukts auf40°C, wurde die Wasserphase (Unterphase) abgetrennt und verworfen. Die organische Oberphase (Pelargonsäurephase) wurde abgekühlt und erstarrte zu einem Kristallbrei. Dieser wurde, wie bei I angegeben, filtriert bzw. abgesaugt und der zurückbleibende Filterkuchen gewonnen.

### Beispiel 5

Der Filterkuchen aus III wurde wie bei I mit kaltem (ca. 20°C) Wasser behandelt und filtriert. Es verblieb ein pelargonfeuchter Filterkuchen (79,2g). Dieser Filterkuchen war Ausgangsmaterial für die Reinigungsoperationen 1. bis 4.

### 1. rohe Azelainsäure

Ein Aliquot (8,1 g ) des gewonnenen Filterkuchens wurde zur Entfernung der Pelargonsäure und leichter flüchtigen Verbindungen einer Vakuumdestillation unterworfen. Es verblieb ein in der Kälte fester Rückstand (rohe Azelainsäure, 4,23g).

Die Neutralisationstitration zur Bestimmung des Azelainsäuregehaltes zeigte einen gegenüber dem Verbrauch an NaOH bei reiner Azelainsäure um 3,9% verminderten Verbrauch an NaOH.

### 2. mit kalter Pelargonsäure gewaschene Azelainsäure

Ein Aliquot (15 g) des Filterkuchens wurde mit kalter (ca. 20°C) Pelargonsäure (35g) innigst verrührt und anschließend wurde mittels Büchnertrichter filtriert.
Ein Aliquot (8,1 g ) des so gewonnenen Filterkuchens wurde zur Entfernung der Pelargonsäure und leichter flüchtigen Verbindungen einer Vakuumdestillation unterworfen. Es verblieb ein sich beim Abkühlen verfestigender Rückstand (gereinigte Azelainsäure, 3,54g ).
Die Neutralisationstitration zur Bestimmung des Azelainsäuregehaltes zeigte einen gegenüber reiner Azelainsäure um 0,77 bis 0,8 % verminderten Verbrauch an NaOH.

### 3. aus Pelargonsäure umkristallisierte Azelainsäure

Ein Aliquot (15 g) des Filterkuchens wurde in Pelargonsäure (40g ) heiß gelöst, anschließend wieder auf Raumtemperatur abgekühlt und die ausfallenden Kristalle mittels Büchnertrichter filtriert. Es verblieben 14,8 g pelargonsäurefeuchter Filterkuchen.
Ein Aliquot (6,97 g ) des so gewonnenen Filterkuchens wurde zur Entfernung der Pelargonsäure und leichterflüchtigen Verbindungen einer Vakuumdestillation unterworfen. Es verblieb ein in der Kälte fester Rückstand (gereinigte Azelainsäure 2,23g ).
Die Neutralisationstitration zur Bestimmung des Azelainsäuregehaltes zeigte einen gegenüber reiner Azelainsäure um 0,9% verminderten Verbrauch an NaOH.

Der Vergleich von Versuch 2. und 3. zeigt, daß die erste Umkristallisation in der Pelargonsäure der oxidativen Ozonolyse mit dem anschließenden Waschvorgang gemäß Versuch 2. bereits eine sehr gute Reinheit liefert.

### 4. mit kalter Pelargonsäure gewaschen und anschließend aus Pelargonsäure umkristallisierte Azelainsäure, Kondensation mit Hexamethylendiamin zu Polyamid

Die verbleibende Restmenge des pelargonfeuchten Filterkuchens wurde mit kalter Pelargonsäure wie in 2. gewaschen. Der so erhaltene Filterkuchen wurde mit den Azelainsäure enthaltenden Fraktionen 1. bis 3. (Filterkuchenreste und feste Rückstände) vereint. Die Gesamtmenge wurde aus Pelargonsäure (120g) umkristallisiert und filtriert. Der gewonnene Filterkuchen wurde zur Entfernung der Pelargonsäure und leichter flüchtigen Verbindungen einer Vakuumdestillation unterworfen.
Die Neutralisationstitration zur Bestimmung des Azelainsäuregehaltes zeigte gegenüber reiner Azelainsäure keinen Minder- noch Mehrverbrauch an NaOH. Das so erhaltene Produkt wurde in bekannter Weise mit Hexamethylendiamin im Autoklav zu Polyamid kondensiert (Endkondensationstemperatur ca. 300°C).
Das entstandene Polyamid weist die erwarteten Eigenschaften auf: es ist farblos, leicht opaleszierend, bei Raumtemperatur zäh und nicht brüchig, kaltreckfähig, äußerst reißfest und thermoplastisch verarbeitbar.

### Beispiel 6

### Reinigung von Brassyl- und Azelainsäure aus der oxidativen Ozonolyse von Eruca/Ölsäuregemischen (Fettsäuren aus Crambeöl, "Crambesäure")

Die Umsetzung erfolgte analog zur oxidativen Ozonolyse, wie unter I aufgeführt, allerdings wurde als Rohstoff Crambesäure (13 Gew.% Ölsäure, 8 Gew.% Linolsäure, 6 Gew.% Linolensäure, 62 Gew.% Erucasäure, und 3 Gew.% Palmitin- und Stearinsäure sowie 8 Gew.% Fettsäuren mit Kettenlängen >C20) eingesetzt.
Nach Abkühlen des heißen Oxidationsprodukts auf Raumtemperatur (ca.20°C) wurde der sich bildende Kristallbrei, wie bei I angegeben, filtriert bzw. abgesaugt und der zurückbleibende Filterkuchen gewonnen. Dieser wurde mit Wasser, wie bei I angegeben, gewaschen und durch Filtration ein Filterkuchen gewonnen. Der Filterkuchen wurde mittels Vakuumdestillation von der Pelargonsäure und leichter flüchtigen Verbindungen befreit. Es wurden ein Destillat (28,3g Pelargonsäure) und ein bei Raumtemperatur fester Rückstand (37,7g rohe Dicarbonsäuremischung) gewonnen. Letzterer war Ausgangspunkt für die nachfolgenden Untersuchungen.

### 1. rohe Dicarbonsäuren

Ein Aliquot der rohen Dicarbonsäuren wurde in an sich bekannter Weise zu Methylestern sauer katalysiert verestert.
Das Gaschromatographie-Spektrum zeigte als Hauptpeaks Azelainsäure- und Brassylsäuredimethylester im erwarteten Verhältnis der Integrationsflächen ihrer Signale von etwa 1 zu 4. Daneben waren Verunreinigungen z.B. die Methylester der Palmitin-, Stearin- und Eicosensäure, deutlich zu sehen und machten ca. 10% der Gesamtintegrationsfläche der Signale aus .

### 2. Umkristallisieren aus Pelargonsäure

Die verbliebene Hauptmenge der rohen Dicarbonsäure (ca. 35 g) wurde aus Pelargonsäure (200 ml) umkristallisiert und die Kristallmasse abfiltriert. Der Filterkuchen wurde durch Vakuumdestillation von der Pelargonsäure befreit. Es wurden 26,4 g Feststoff (gereinigte Dicarbonsäuremischung) gewonnen. Ein Aliquot der so gereinigten Dicarbonsäuren wurde in an sich bekannter Weise zu Methylestern sauer katalysiert verestert. Das Gaschromatographie-Spektrum der Methylester zeigte als Hauptpeaks Azelain- und Brassylsäuredimethylester. Der Integrationsanteil der Signale der Palmitin-, Stearin- und Eicosensäuremethylester hatten im Vergleich zum Spektrum der Methylester der rohen Dicarbonsäuremischungen deutlich abgenommen (um etwa den Faktor 10).

## Patentansprüche

1. Verfahren zur Gewinnung von gesättigten Dicarbonsäuren der Kettenlänge C6 bis C21 bzw. entsprechenden diamidischen Dicarbonsäuren aus einer Fettsäurespaltung ungesättigter Fettsäuren bzw. der Bis-Fettsäurediamide dieser ungesättigten Fettsäuren durch oxidative Ozonolyse und nachfolgende Abtrennung und Aufreinigung der Dicarbonsäuren,
**dadurch gekennzeichnet**,
a) daß nach der oxidativen Ozonolyse deren Reaktionsprodukte in einer Carbonsäure oder einer Mischung mehrerer Carbonsäuren mittlerer Kettenlänge von C6 bis C12 oder von Estern kurzkettiger Alkohole dieser Carbonsäure(n) als Umkristallisationslösemittel in der Hitze gelöst werden,
b) daß die Lösung auf eine Temperatur abgekühlt wird, bei der die Dicarbonsäuren möglichst weitgehend auskristallisiert sind, jedoch das Umkristallisationslösemittel die übrigen Reaktionsprodukte der oxidativen Ozonolyse und anderen Verunreinigungen noch in gelöster Form enthält und selbst nicht ausfällt, und
daß die auskristallisierten Dicarbonsäuren anschließend dadurch gewonnen werden,
c1 ) daß sie im noch abgekühlten Zustand in Form eines Kristallbreis abgetrennt werden, der noch Reste an dem Umkristallisationslösemittel mit den darin gelösten Bestandteilen enthält,
c2) der Kristallbrei gegebenenfalls nach weiteren Umkristallisationsschritten mit einem entsprechend abgekühlten Waschlösemittel oder einer Waschlösemittelmischung gewaschen wird und
c3) die Waschlösemittelreste anschließend entfernt werden.

2. Verfahren nach Anspruch 1,
**dadurch gekennzeichnet,**
**daß** als die ungesättigten Fettsäuren Fettsäuren oder Fettsäuremischungen nativen Ursprungs der Kettenlänge C14 bis C24, vorzugsweise als einfach ungesättigte Fettsäuren eingesetzt werden, sowie Fettsäuren oder Fettsäuremischungen, vorzugsweise nativen Ursprungs, eingesetzt werden, die durch Verseifen von Ölen, insbesondere High-Oleic-Ölen, erhältlich sind, welche durch Teilhydrierung von mehrfach ungesättigten Fettsäuren befreit sind.

3. Verfahren nach Anspruch 1 oder 2,
**dadurch gekennzeichnet,**
**daß** als das Umkristallisationslösemittel die bei der oxitativen Ozonolyse entstehende und vorzugsweise auch als Reaktionslösemittel der oxidativen Ozonolyse verwendete Monocarbonsäure, insbesondere die Pelargonsäure, genutzt wird, um die übrigen Reaktionsprodukte der Ozonolyse in der Hitze bei vorzugsweise nicht mehr als 100 °C zu lösen, wobei bevorzugt die vollständige Reaktionslösung der oxidativen Ozonolyse einschließlich des bei der oxidativen Ozonolyse zugesetzten Wassers im Verfahrensschritt a) eingesetzt wird.

4. Verfahren nach einem der vorhergehenden Ansprüche,
**dadurch gekennzeichnet,**
**daß** als Fettsäureausgangsprodukt der oxidativen Ozonolyse a) Fettsäuremischungen eingesetzt werden, deren Anteil gesättigter Fettsäuren mit Kettenlängen größer C14 und kleiner C26 unter 15 Gew.% , vorzugsweise unter 10 Gew.%, der zu ozonisierenden Fettsäuremischung liegt, und b) Fettsäurediamidmischungen eingesetzt werden, bei denen die in den Fettsäurediamiden gebundenen gesättigten Fettsäuren weniger als 15 Gew.%, vorzugsweise weniger als 10 Gew.% des Gesamtanteils der Fettsäuren ausmachen, und daß die oxidative Ozonolyse in an sich bekannter Weise so durchgeführt wird, daß die Ausbeute an der Dicarbonsäure bzw. diamidischen Dicarbonsäure mindestens 85 Gew.% der theoretisch zu erwartenden Ausbeute beträgt.

5. Verfahren nach einem der vorhergehenden Ansprüche,
**dadurch gekennzeichnet,**
**daß** die eingesetzte Gewichtsmenge des Umkristallisationslösemittels im Fall nichtdiamidischer Dicarbonsäuren das ein- bis zehnfache und im Fall diamidischer Dicarbonsäuren das zwei- bis fünfzehnfache der Gewichtsmenge des Reaktionsendprodukts der oxidativen Ozonolyse abzüglich der Gewichtsmenge des darin enthaltenen Reaktionslösemittels beträgt.

6. Verfahren nach einem der vorhergehenden Ansprüche,
**dadurch gekennzeichnet,**
**daß** die Kristallisationslösung im Schritt a) und b) so eingestellt wird, daß die Kristallisation aus einer bei circa 80 °C bis 100 °C gesättigten Lösung erfolgt und die Lösung auf unter 25 °C, vorzugsweise auf etwa 15 °C, abgekühlt wird.

7. Verfahren nach einem der vorhergehenden Ansprüche,
**dadurch gekennzeichnet,**
**daß** als Waschlösemittel kalte Lösemittel einer Temperatur unter 25 °C und bevorzugt von 15°C, in Form von Fettsäuren mit einer Kettenlänge C6 bis C12 sowie Estern dieser Fettsäuren und Mischungen solcher Fettsäuren und/oder Fettsäureestern eingesetzt werden und daß die Waschlösemittelreste vorzugsweise destillativ entfernt werden.

8. Verfahren nach einem der vorhergehenden Ansprüche,
**dadurch gekennzeichnet,**
**daß** die Waschlösemittelreste erst dann entfernt werden, wenn durch mehrmaliges Zusetzen einer bestimmten Waschlösemittelmenge oder durch sukzessives Durchdrücken einer Waschlösemittelsäule durch den Kristallbrei die am Kristallbrei anhaftenden Umkristallisationslösemittelreste im wesentlichen durch Waschlösemittel ersetzt sind.

9. Verfahren nach einem der vorhergehenden Ansprüche,
**dadurch gekennzeichnet,**
**daß** im Anschluß an den Verfahrensschritt c1) unter Auslassung der Schritte c2) und c3), vorzugsweise jedoch im Anschluß an den Verfahrensschritt c3), die gewonnenen nicht-diamidischen Dicarbonsäuren mit Diaminen in einem Reaktionslösemittel, vorzugsweise in Form eines Alkohols, in die Ammoniumsalze der Diamine umgesetzt werden, aus denen anschließend die Dicarbonsäuren in vollständig reiner Form wieder freigesetzt werden.

10. Verfahren nach einem der Ansprüche 1 bis 8,
**dadurch gekennzeichnet,**
**daß** im Anschluß an den Verfahrensschritt c1) unter Auslassung der Schritte c2) und c3), vorzugsweise jedoch im Anschluß an den Verfahrensschritt c3), die gewonnenen diamidischen Dicarbonsäuren mit Diaminen in einem Reaktionslösemittel, vorzugsweise in Form eines Alkohols, in die Ammoniumsalze der Diamine umgesetzt werden, die entweder für die Polyamidsynthese eingesetzt werden oder aus denen anschließend die diamidischen Dicarbonsäuren in vollständig reiner Form wieder freigesetzt werden.

11. Verfahren nach Anspruch 9 oder 10,
**dadurch gekennzeichnet,**
**daß** der Alkohol in Form eines wasserlöslichen Alkohols wie Methanol, Ethanol, Propanol in der fünf- bis zehnfachen Gewichtsmenge der Gewichtsmenge an Dicarbonsäure einschließlich der hierzu etwa stöchiometrischen Menge an Diaminen zugegeben wird.

12. Verfahren nach einem der Ansprüche 9 bis 11,
**dadurch gekennzeichnet,**
**daß** das salzbildende Diamin so ausgewählt wird, daß mit ansteigender Kettenlänge der Dicarbonsäure oder diamidischen Dicarbonsäure niedermolekularere Diamine eingesetzt werden, um über die damit beeinflußte Löslichkeit des sich bildenden Salzes eine selektive Auskristallisation der Salze der Dicarbonsäuren zu begünstigen.

13. Verfahren nach einem der Ansprüche 9 bis 12,
**dadurch gekennzeichnet,**
**daß** die mit dem Alkohol versetzten Diamine und Dicarbonsäuren bis zum Siedepunkt des Alkohols erhitzt werden und, falls keine klare Lösung entsteht, durch Zusatz von Wasser die Löslichkeit des Salzes soweit verschoben wird, bis die klare Lösung entsteht,
**daß** der PH-Wert der klaren Lösung durch Zugabe von roher Dicarbonsäure oder Diamin auf circa 7,5 eingestellt wird und
nach Abkühlen der Lösung die auskristallisierten Salze der Diamine abgetrennt werden.

14. Verfahren nach einem der Ansprüche 9 bis 13,
**dadurch gekennzeichnet,**
**daß** aus den Salzen der Diamine die Dicarbonsäuren oder diamidischen Dicarbonsäuren unter Zugabe von verdünnten Mineralsäuren freigesetzt werden.

## Claims

1. A method of producing saturated dicarboxylic acids with a chain length of C6 to C21 or the corresponding diamidic dicarboxylic acids, respectively, from fatty acid cleavage of unsaturated fatty acids or the bis-fatty acids of said unsaturated fatty acids, respectively, by oxidative ozonolysis and subsequent separation and purification of the dicarboxylic acids, **characterized by**:
a) dissolving, after oxidative ozonolysis, the reaction products thereof under heating in a carboxylic acid or in a mixture of a plurality of carboxylic acids of a medium chain length of C6 to C12 or of esters of short-chain alcohols of these carboxylic acid(s) as recrystallization solvent,
b) cooling the solution to a temperature at which the dicarboxylic acids crystallize out as much as possible, but the recrystallization solvent contains the other reaction products of oxidative ozonolysis and other impurities still in dissolved form and does not precipitate itself, and then
obtaining the crystallized dicarboxylic acids by
c1) separating them while still cool in form of a crystal sludge which still contains residues of the recrystallization solvent with the dissolved components in it,
c2) washing the crystal sludge with a correspondingly cooled wash solvent or a wash solvent mixture, optionally after additional recrystallization steps, and
c3) subsequently removing the wash solvent residues.

2. The method according to claim 1,
**characterized in that**, as said unsaturated fatty acids, fatty acids or fatty acids mixtures of native origin with a chain length of C14 to C24, preferably in form of monounsaturated fatty acids, as well as fatty acids or fatty acid mixtures, preferably of native origin, are used, which are obtainable by saponification of oils, in particular high-oleic oils, which are freed of polyunsaturated fatty acids by partial hydrogenation.

3. The method according to claim 1 or 2,
**characterized in that** a monocarboxylic acid, in particular pelargonic acid, produced in the oxidative ozonolysis and preferably also used as the reaction solvent in the oxidative ozonolysis is used as the recrystallization solvent to dissolve the other reaction products of ozonolysis at high temperatures of preferably no more than 100 °C, preferably with the entire reaction solution of oxidative ozonolysis, including the water added in oxidative ozonolysis, being used in process step a).

4. The method according to any preceding claim,
**characterized in that** a) fatty acid mixtures in which the saturated fatty acid content with chain lengths greater than C14 and smaller than C26 amounts to less than 15 wt%, preferably less than 10 wt% of the fatty acid mixture to be ozonized, are used as the fatty acid starting material for oxidative ozonolysis, and b) fatty acid diamide mixtures where the saturated fatty acids bound in the fatty acid diamides constitute less than 15 wt%, preferably less than 10 wt% of the total fatty acid content, are used as the fatty acid starting material for oxidative ozonolysis, and that the oxidative ozonolysis is carried out in a manner known per se, so that the yield of dicarboxylic acid or diamidic dicarboxylic acid amounts to at least 85 wt% of the yield to be expected theoretically.

5. The method according to any preceding claim,
**characterized in that** the starting amount by weight of the recrystallization solvent is one to ten times in case of non-diamidic dicarboxylic acids and two to fifteen times in case of diamidic dicarboxylic acids of the amount by weight of the reaction end product of oxidative ozonolysis minus the amount by weight of the reaction solvent present in the reaction end product.

6. The method according to any preceding claim,
**characterized in that** the crystallization solution in steps a) and b) is adjusted such that crystallization takes place from a solution that is saturated at approximately 80 °C to 100 °C, and the solution is cooled to a temperature below 25 °C, preferably to approximately 15 °C.

7. The method according to any preceding claim,
**characterized in that** cold solvents at a temperature below 25 °C, preferably 15 °C, in the form of fatty acids with a chain length of C6 to C12 and esters of these fatty acid and mixtures of such fatty acids and/or fatty acid esters are used as the wash solvent, and that the residues of wash solvent are preferably removed by distillation.

8. The method according to any preceding claim,
**characterized in that** the wash solvent residues are only removed when the recrystallization solvent residues adhering to the crystal sludge have been substantially replaced by wash solvent by repeatedly adding a certain amount of wash solvent or by successively forcing a wash solvent column through the crystal sludge.

9. The method according to any preceding claim,
**characterized in that** following process step c1), under omitting steps c2) and c3), but preferably following process step c3), the non-diamidic dicarboxylic acids thus obtained are reacted with diamines in a reaction solvent, preferably in the form of an alcohol, to convert them to the ammonium salts of the diamines, from which the dicarboxylic acids are then released in completely pure form.

10. The method according to any of claims 1 to 8,
**characterized in that** following process step c1), under omitting steps c2) and c3), but preferably following process step c3), the diamidic dicarboxylic acids thus obtained are reacted with diamines in a reaction solvent, preferably in the form of an alcohol, to form the ammonium salts of the diamines, which are either used for polyamide synthesis or from which the diamidic dicarboxylic acids are then released in completely pure form.

11. The method according to 9 or 10,
**characterized in that** the alcohol is added in form of a water-soluble alcohol such as methanol, ethanol or propanol in a five-fold to ten-fold amount by weight of the total amount by weight of dicarboxylic acid including an approximately stoichiometric amount of diamines.

12. The method according to any of claims 9 to 11,
**characterized in that** the salt-forming diamine is selected so that more lower molecular diamines are used with increasing chain length of the dicarboxylic acid or diamidic dicarboxyilc acid to promote a selective crystallization of the salts of the dicarboxylic acids by the resulting influence on the solubility of the salt being formed.

13. The method according to any of claims 9 to 12,
**characterized in that** the diamines and dicarboxylic acids mixed with the alcohol are heated to the boiling point of the alcohol and if a clear solution is not formed, the solubility of the salt is shifted by adding water until a clear solution is obtained,
that the pH of the clear solution is adjusted to approximately 7.5 by adding crude dicarboxylic acid or diamine, and
that, after cooling the solution, the salts of the diamines that crystallize out are isolated.

14. The method according any of claims 9 to 13,
**characterized in that** the dicarboxylic acids or diamidic dicarboxylic acids are released from the salts of the diamines by adding dilute mineral acids.

## Revendications

1. Procédé d'obtention d'acides dicarboxyliques saturés d'une longueur de chaîne C6 à C21 ou d'acides dicarboxyliques diamidiques correspondants à partir d'une fission d'acides gras insaturés ou des bis-diamides d'acides gras de ces acides gras insaturés par ozonolyse oxydante suivie d'une séparation et d'une purification des acides dicarboxyliques,
**caractérisé en ce que**
a) après l'ozonolyse oxydante, ses produits réactionnels sont dissous à la chaleur dans un acide carboxylique ou un mélange de plusieurs acides carboxyliques de longueur de chaîne moyenne de C6 à C12 ou d'esters d'alcools à chaîne courte de cet(s) acide(s) carboxylique(s) en tant que dissolvant de recristallisation,
b) la solution est refroidie à une température à laquelle les acides dicarboxyliques sont cristallisés autant que possible, mais le dissolvant de recristallisation contient encore sous forme dissoute les autres produits réactionnels de l'ozonolyse oxydante et d'autres impuretés et ne précipite pas lui-même, et
que les acides dicarboxyliques cristallisés sont ensuite obtenus en
c1) les séparant à l'état encore refroidi sous forme d'une bouillie cristalline qui contient encore des résidus de dissolvant de recristallisation avec les composantes dissoutes dedans,
c2) lavant la bouillie cristalline le cas échéant après d'autres étapes de recristallisation à l'aide d'un dissolvant nettoyant refroidi en conséquence ou d'un mélange de dissolvants nettoyants et
c3) éliminant ensuite les résidus de dissolvant nettoyant.

2. Procédé selon la revendication 1,
**caractérisé en ce que**
que sont utilisés en tant qu'acides gras insaturés acides gras ou mélanges d'acides gras d'origine de longueur de chaîne C14 à 24, de préférence des acides gras à insaturation simple, ainsi que des acides gras ou mélanges d'acides gras, de préférence d'origine, qui peuvent être obtenus par saponification d'huiles, notamment d'huiles hautement oléïques qui sont débarrassées par hydrogénation partielle des acides gras à insaturation multiple.

3. Procédé selon la revendication 1 ou 2,
**caractérisé en ce que**
est utilisé comme dissolvant de recristallisation l'acide monocarboxylique apparaissant lors de l'ozonolyse oxydante et utilisé de préférence aussi comme dissolvant réactionnel de l'ozonolyse oxydante, notamment l'acide pélargonique, afin de dissoudre les autres produits réactionnels de l'ozonolyse à la chaleur, de préférence pas plus de 100 °C, la solution réactionnelle complète de l'ozonolyse oxydante, y compris de l'eau ajoutée lors de l'ozonolyse oxydante, étant mise en oeuvre au cours de l'étape a) du procédé.

4. Procédé selon une des revendications précédentes,
**caractérisé en ce que**
sont utilisés comme produit de base aux acides gras de l'ozonolyse oxydante a) des mélanges d'acides gras qui contiennent une quantité d'acides gras saturés de longueurs de chaîne supérieures à C14 et inférieures à C26 représentant moins de 15 % en poids, de préférence moins de 10 % en poids, du mélange d'acides gras à ozonolyser, et b) des mélanges de diamides d'acides gras dans lesquels les acides gras saturés liés dans les diamides d'acides gras représentent moins de 15 % en poids, de préférence moins de 10 % en poids, de la quantité totale d'acides gras, et que l'ozonolyse oxydante est réalisée de manière connue en soi de manière à ce que le rendement en acide dicarboxylique ou en acide dicarboxylique diamidique soit d'au moins 85 % en poids du rendement à attendre théoriquement.

5. Procédé selon une des revendications précédentes,
**caractérisé en ce que**
la quantité en poids utilisée de dissolvant de recristallisation est, en cas d'acides dicarboxyliques non diamidiques, de une à dix fois et, en cas d'acides dicarboxyliques diamidiques, de deux à quinze fois la quantité en poids du produit réactionnel de l'ozonolyse oxydante, moins la quantité en poids du dissolvant réactionnel contenu dedans.

6. Procédé selon une des revendications précédentes,
**caractérisé en ce que**
la solution de recristallisation est définie dans les étapes a) et b) de manière à ce que la cristallisation ait lieu à partir d'une solution saturée à environ 80 °C à 100 °C et que la solution soit refroidie à moins de 25 °C, de préférence à environ 15 °C.

7. Procédé selon une des revendications précédentes,
**caractérisé en ce que**
sont utilisés comme dissolvants nettoyants des dissolvants froids d'une température inférieure à 25 °C et de préférence de 15 °C, sous forme d'acides gras d'une longueur de chaîne C6 à C12 ainsi que d'esters de ces acides gras et de mélanges de tels acides gras et/ou d'esters d'acides gras et que les résidus de dissolvant nettoyant sont de préférence éliminés par distillation.

8. Procédé selon une des revendications précédentes,
**caractérisé en ce que**
les résidus de dissolvant nettoyant ne sont éliminés que lorsque, du fait d'un ajout à plusieurs reprises d'une certaine quantité de dissolvant nettoyant ou par introduction successive d'une colonne de dissolvant nettoyant à travers la bouillie cristalline, les résidus de dissolvant de recristallisation adhérant à la bouillie cristalline sont substantiellement remplacés par du dissolvant nettoyant.

9. Procédé selon une des revendications précédentes,
**caractérisé en ce que**
consécutivement à l'étape c1) du procédé, en omettant les étapes c2) et c3), mais de préférence consécutivement à l'étape c3) du procédé, les acides dicarboxyliques non diamidiques obtenus sont convertis avec des diamines dans un dissolvant réactionnel, de préférence sous forme d'un alcool, en sels d'ammonium des diamines, à partir desquels sont ensuite de nouveau libérés les acides dicarboxyliques sous forme totalement pure.

10. Procédé selon une des revendications 1 à 8,
**caractérisé en ce que**
consécutivement à l'étape c1) du procédé, en omettant les étapes c2) et c3), mais de préférence consécutivement à l'étape c3) du procédé, les acides dicarboxyliques diamidiques obtenus sont convertis avec des diamines dans un dissolvant réactionnel, de préférence sous forme d'un alcool, en sels d'ammonium des diamines, qui sont soit utilisés pour la synthèse de polyamide, soit à partir desquels sont ensuite de nouveau libérés les acides dicarboxyliques sous forme totalement pure.

11. Procédé selon la revendication 9 ou 10,
**caractérisé en ce que**
l'alcool est rajouté sous forme d'un alcool soluble à l'eau comme le méthanol, l'éthanol, le propanol en une quantité en poids représentant cinq à dix fois la quantité en poids d'acide dicarboxylique, y compris la quantité approximativement stoechiométrique de diamines.

12. Procédé selon une des revendications 9 à 11,
**caractérisé en ce que**
le diamine formant du sel est choisi de manière à ce que qu'au fur et à mesure que la longueur de chaîne de l'acide dicarboxylique ou de l'acide dicarboxylique diamidique augmente, on utilise des diamines plus faiblement moléculaires, afin de favoriser, grâce à la solubilité ainsi influencée du sel se formant, une cristallisation sélective des sels des acides dicarboxyliques.

13. Procédé selon une des revendications 9 à 12,
**caractérisé en ce que**
les diamines et acides dicarboxyliques transposés avec l'alcool sont chauffés jusqu'au point d'ébullition de l'alcool et, si l'on n'obtient pas de solution claire, que la solubilité du sel est retardée par ajout d'eau jusqu'à ce que la solution claire apparaisse,la valeur PH de la solution claire est définie en ajoutant de l'acide dicarboxylique brut ou du diamine à environ 7,5 et
les sels cristallisés sont séparés des diamines après le refroidissement de la solution.

14. Procédé selon une des revendications 9 à 13,
**caractérisé en ce que**
les acides dicarboxyliques ou acides dicarboxyliques diamidiques sont libérés à partir des sels de diamines en ajoutant des acides minéraux dilués.
